# EUROPÄISCHE PATENTANMELDUNG

(11) **EP 1 950 271 A1**
(43) Veröffentlichungstag der Anmeldung: **30.07.2008**
(21) Anmeldenummer: 08000686.9
(22) Anmeldetag: 15.01.2008
(51) Int. Cl.: C10G 70/06, C07C 4/20, C07C 7/11

(54) **Verfahren zur trennung der gasförmigen Reaktionsprodukte der Dampf-Dealkylierung**

(30) Priorität: 26.01.2007 DE 102007004079
(71) Anmelder: Linde Aktiengesellschaft, 80807 München (DE)
(72) Erfinder: Göke, Volker, 82515 Wolfrathausen (DE); Neuendorf, Stephanie, 82069 Hohenschäftlarn (DE); Ponceau, Marianne, 81371 München (DE); Schödel, Nicole, 81477 München (DE); Wenning, Ulrike, 82049 Pullach (DE)

(57) **Zusammenfassung**

Die Erfindung beschreibt ein Verfahren zur Trennung der gasförmigen Reaktionsprodukte der Dampf-Dealkylierung einer Kohlenwasserstofffraktion in einer Anlage zur Erzeugung von Kohlenwasserstoffen aus kohlenwasserstoffhaltigem Einsatz, wobei als Reaktionsprodukte der Dampf-Dealkylierung aromatische Kohlenwasserstoffe und entsprechende Alkane neben gasförmigen Reaktionsprodukten vorwiegend bestehend aus Wasserstoff, Kohlenmonoxid, Kohlendioxid und Methan entstehen. Die Trennung der gasförmigen Reaktionsprodukte erfolgt mittels einer Flüssig-Gas Extraktion, bei der mindestens eine ionische Flüssigkeit im Extraktionsmittel enthalten ist oder das Extraktionsmittel aus mindestens einer ionischen Flüssigkeit besteht. Die jeweilige Kohlenwasserstofffraktion (1) wird über einen Wärmetauscher (2) als Einsatzstoff in ein Verfahren zur Dampf-Dealkylierung (3) geführt. Die Reaktionsprodukte der Dampf-Dealkylierung (4) werden über den Wärmetauscher (2), der gleichzeitig die Kohlenwasserstofffraktion (1) vorwärmt, und einen weiteren Kühler (nicht dargestellt) abgekühlt und zu einem 3-Phasenseparator (5) geführt. Dort werden die gasförmigen Reaktionsprodukte (6) von den Kohlenwasserstoffen (7) und Wasser (8) getrennt. Aus den Kohlenwasserstoffen (7) wird das gewünschte Wertprodukt Benzol (9) abgetrennt, während die restlichen Kohlenwasserstoffe (10) ebenso wie das Wasser (8) erneut in den Einsatz zur Dampf-Dealkylierung gefahren werden. Die gasförmigen Reaktionsprodukte werden in ein Verfahren zur Abtrennung von Kohlendioxid mittels Flüssig-Gas-Extraktion mit einer ionischen Flüssigkeit als Extraktionsmittel geführt. Die ionische Flüssigkeit wird mit Kohlendioxid beladen (11) und anschließend zur Regeneration (12) geführt. Dort wird die ionische Flüssigkeit erwärmt, so dass Kohlendioxid aus dem Extraktionsmittel entweicht und als Produkt aus der Anlage geführt wird (13). Die regenerierte Flüssigkeit (14) wird zurück zur Beladung geführt. Die nunmehr kohlendioxidfreien, überwiegend Kohlenmonoxid, Wasserstoff und Methan enthaltenden gasförmigen Reaktionsprodukte (15) werden zu einem Verfahren zur Abtrennung von Wasserstoff geführt. Als Wertprodukte werden somit aus den gasförmigen Reaktionsprodukten Kohlendioxid und Wasserstoff gewonnen.

## Beschreibung

Die Erfindung betrifft Verfahren zur Trennung der gasförmigen Reaktionsprodukte der Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei als Reaktionsprodukte der Dampf-Dealkylierung aromatische Kohlenwasserstoffe und entsprechende Alkane neben gasförmigen Reaktionsprodukten vorwiegend bestehend aus Wasserstoff, Kohlenmonoxid, Kohlendioxid und Methan entstehen.

In einer Anlage zur Erzeugung von Kohlenwasserstoffen aus kohlenwasserstoffhaltigem Einsatz entsteht ein Gemisch aus Kohlenwasserstoffen mit einer unterschiedlichen Anzahl von Kohlenstoffatomen und unterschiedlichen molekularen Konfigurationen. Dieses Gemisch wird in der Anlage zur Erzeugung von Kohlenwasserstoffen aus kohlenwasserstoffhaltigem Einsatz in die einzelnen Kohlenwasserstoffprodukte aufgetrennt.

Abhängig von der jeweiligen Trennsequenz und der Art der Anlage zur Erzeugung von Kohlenwasserstoffen aus kohlenwasserstoffhaltigem Einsatz entstehen eine Kohlenwasserstofffraktion mit mindestens sechs Kohlenstoffatomen (C₆₊-Fraktion) und/oder eine Kohlenwasserstofffraktion mit mindestens sieben Kohlenstoffatomen (C₇₊-Fraktion). Diese Fraktionen enthalten als wirtschaftlich verwertbares Produkt Aromaten, welche als Ausgangsstoff für die Synthese zahlreicher Kunststoffe und zur Erhöhung der Klopffestigkeit von Benzin Verwendung finden.

Um an die wirtschaftlich verwertbaren Produkte der C₆₊-Fraktion, vor allem Benzol, zu gelangen und die Ausbeute möglichst maximal zu gestalten, wird nach dem Stand der Technik folgendes Verfahren angewendet. Die C₆₊-Fraktion wird in eine Fraktion überwiegend bestehend aus Kohlenwasserstoffen mit sechs Kohlenstoffatomen (C₆-Fraktion) und eine C₇₊-Fraktion getrennt. Aus der C₆-Fraktion kann direkt das wirtschaftlich verwertbare Produkt Benzol abgetrennt werden. Aus der C₇₊-Fraktion werden mittels einer Flüssig-Flüssig Extraktion die linearen Kohlenwasserstoffe abgetrennt und als so genanntes Raffinat weiterverarbeitet. Die von den linearen Kohlenwasserstoffen befreite C₇₊-Fraktion enthält nunmehr hauptsächlich Aromaten mit sieben bis acht Kohlenstoffatomen und wird in eine Fraktion überwiegend bestehend aus Kohlenwasserstoffen mit sieben Kohlenstoffatomen (hauptsächlich Toluol) und in eine Fraktion überwiegend bestehend aus Kohlenwasserstoffen mit acht Kohlenstoffatomen (hauptsächlich Xylol) getrennt. Die Fraktion überwiegend bestehend aus Kohlenwasserstoffen mit acht Kohlenstoffatomen wird als Einsatzstoff in ein Verfahren zur para-Xylol Gewinnung geführt. Die Fraktion überwiegend bestehend aus Kohlenwasserstoffen mit sieben Kohlenstoffatomen wird als Einsatz in ein Verfahren zur Hydro-Dealkylierung geführt wird.

Ein derartiges Verfahren zur Hydro-Dealkylierung ist zum Beispiel in WO2005071045 beschrieben. Die Kohlenwasserstoffe werden in Anwesenheit eines Katalysators bei einer Temperatur von 400°C bis 650°C und einem Druck zwischen 20 bar und 40 bar mit Wasserstoff kontaktiert, wobei der Wasserstoff in einem molaren Überschuss vom drei- bis sechsfachen der Kohlenwasserstoffe vorliegt. Unter diesen Bedingungen werden die Alkylgruppen von den jeweiligen alkylierten Aromaten (wie zum Beispiel Toluol) abgespalten, so dass sich Benzol und die jeweiligen Alkane (zum Beispiel Methan) bilden.

Der Verbrauch von Wasserstoff bei der Hydro-Dealkylierung der Kohlenwasserstoffe wirkt sich negativ auf die Wirtschaftlichkeit dieses Verfahrens nach dem Stand der Technik zur Benzolgewinnung aus.

In einem alternativen Verfahren werden die C₆₊-Fraktion und/oder die C₇₊-Fraktion einer Dampf-Dealkylierung unterzogen (siehe zum Beispiel eingereichte Patentschriften DE102006058528.3 oder DE102006058529.1). Bei einer Dampf-Dealkylierung der C₆₊-Fraktion und/oder C₇₊-Fraktion entstehen hauptsächlich die beiden verwertbaren Produktstoffe Benzol und Wasserstoff neben Reaktionsprodukten wie Kohlenmonoxid und Kohlendioxid. Als Einsatzstoff der Dampf-Dealkylierung wird somit lediglich kostengünstiger Wasserdampf bei gleichzeitiger Produktion des gewünschten Wertproduktes Benzol und des wertvollen Nebenproduktes Wasserstoff benötigt.

In diesem Verfahren nach dem Stand der Technik reagieren die Kohlenwasserstoffe aus der jeweiligen Kohlenwasserstofffraktion mit Wasserdampf in der Gasphase an einem festen Katalysator. Dabei werden die Kohlenwasserstoffe und der Wasserdampf in einer Vorrichtung, zum Beispiel Rohre, an dem festen Katalysator vorbeigeführt, wobei sich der Katalysator im Inneren der Vorrichtung, zum Beispiel im Rohrinneren, befindet. Die für die Dealkylierung nötige Reaktionswärme wird der Vorrichtung von außen zugeführt, zum Beispiel durch Verbrennung der gasförmigen Reaktionsnebenprodukte Kohlenmonoxid und Methan mit Luft. Nach dem Stand der Technik entspricht die Temperatur der Einsatzstoffe der Temperatur der Reaktionsprodukte und liegt im Bereich von 400°C bis 600°C. Das Katalysatormaterial im Inneren der Vorrichtung besteht im Wesentlichen aus einem porösen Trägermaterial wie γ-Al₂O₃, MgAl Spinell und/oder Cr₂O₃, und einer an der Oberfläche des Trägermaterials befindlichen Aktivkomponente, wie Rh mit 0.1-1.0 Gew% Beladung und/oder Pd mit 0.2-2.0 Gew% Beladung. Aus den gasförmigen Reaktionsprodukten der Dampf-Dealkylierung wird über eine Druckwechseladsorption mit vorheriger Verdichtung der wirtschaftlich verwertbare Wasserstoff von den Reaktionsnebenprodukten wie Kohlenmonoxid, Kohlendioxid und Methan getrennt.

Nach dem Stand der Technik wird von den gasförmigen Reaktionsprodukten nur das Wertprodukt Wasserstoff abgetrennt, während der Rest der gasförmigen Reaktionsprodukte als Brenngas Verwendung findet.

Der Erfindung liegt daher die Aufgabe zugrunde, ein Verfahren der eingangs beschriebenen Art derart auszugestalten, dass eine einfache Auftrennung der gasförmigen Reaktionsprodukte erreicht und die Verwertung der gesamten gasförmigen Reaktionsprodukte optimiert wird.

Die gestellte Aufgabe wird dadurch gelöst, dass die Trennung der gasförmigen Reaktionsprodukte mittels Flüssig-Gas Extraktion erfolgt, wobei als Extraktionsmittel eine Flüssigkeit eingesetzt wird, die zumindest eine ionische Flüssigkeit enthält oder aus einer oder mehreren ionischen Flüssigkeiten besteht und das Extraktionsmittel zuerst mit der herauszutrennenden Komponente beladen und anschließend unter Freisetzung der herauszutrennenden Komponente regeneriert wird.

lonische Flüssigkeiten sind niederschmelzende, organische Salze mit Schmelzpunkten zwischen -90°C und 100°C, wobei die meisten der bekannten ionischen Flüssigkeiten bereits bei Raumtemperatur in flüssiger Form vorliegen. Im Gegensatz zu herkömmlichen, molekularen Flüssigkeiten sind ionische Flüssigkeiten zur Gänze ionisch und zeigen deshalb neue und ungewöhnliche Eigenschaften. lonische Flüssigkeiten sind durch die Variation der Struktur von Anion und/oder Kation sowie durch die Variation von deren Kombinationen in ihren Eigenschaften an gegebene technische Problemstellungen vergleichsweise gut anpassbar. So können beispielsweise Dichte und das Mischungsverhalten mit anderen Stoffen bei ionischen Flüssigkeiten durch die Wahl der Ionen in weiten Grenzen beeinflusst bzw. eingestellt werden. Aus diesem Grund werden sie oftmals auch als so genannte "Designer Solvents" bezeichnet. Bei herkömmlichen, molekularen Flüssigkeiten ist hingegen lediglich eine Variation der Struktur möglich.

Im Gegensatz zu konventionellen, molekularen Flüssigkeiten haben ionische Flüssigkeiten darüber hinaus den Vorteil, dass sie keinen messbaren Dampfdruck besitzen. Dies bedeutet, dass sie - solange ihre Zersetzungstemperatur, die oftmals oberhalb von 250°C liegt, nicht erreicht wird - selbst im Hochvakuum nicht in geringsten Spuren verdampfen. Die Regenerierung einer als Extraktionsmittel verwendeteten ionischen Flüssigkeit ist daher durch Temperaturerhöhung und/oder Druckabsenkung nahezu vollständig möglich, ohne dass hierbei Extraktionsmittel verloren geht. Gleichzeitig kann das Extrakt im Zuge der Regenerierung mit extrem hoher Reinheit gewonnen werden.

Vorzugsweise werden zur Durchführung des erfindungsgemäßen Verfahrens Extraktionsmittel eingesetzt, die ionische Flüssigkeiten enthalten, die aus der Menge der im Unteranspruch 2 aufgelisteten ionischen Flüssigkeiten gewählt werden.

Vorteilhafterweise erfolgt die Trennung der gasförmigen Reaktionsprodukte im Gleichstrom oder im Gegenstrom mit dem Extraktionsmittel oder durch Durchperlen des Extraktionsmittels. Die Regeneration des Extraktionsmittels erfolgt zweckmäßigerweise mittels Druckreduktion, Anlegung eines Unterdrucks oder Vakuums und/oder mittels Temperaturerhöhung des Extraktionsmittels.

In einer bevorzugten Ausgestaltung des erfindungsgemäßen Verfahrens werden mindestens zwei Vorrichtungen zur Beladung und Regeneration verwendet, wobei immer in mindestens einer Vorrichtung die Beladung und in mindestens einer Vorrichtung die Regeneration des Extraktionsmittels erfolgt. Durch die Verwendung von mindestens zwei Vorrichtungen zur Beladung und Regeneration wird eine kontinuierliche Trennung der gasförmigen Reaktionsprodukte gewährleistet. Je nach gewählter Verfahrensweise kann in einer Vorrichtung immer die Beladung des Extraktionsmittels und in der zweiten Vorrichtung die Regeneration des Extraktionsmittels erfolgen oder Beladung und Regeneration erfolgen in der gleichen Vorrichtung, wobei mindestens eine weitere Vorrichtung zur Beladung und Regeneration antizyklisch zur ersten Vorrichtung betrieben wird.

In einer Ausgestaltung der Erfindung werden mindestens zwei Verfahren zur Flüssig-Gas Extraktion nacheinander unter Verwendung unterschiedlich zusammengesetzter Extraktionsmittel durchgeführt. Durch die aufeinanderfolgende Anwendung des erfindungsgemäßen Verfahrens mit unterschiedlichen Extraktionsmitteln läßt sich die Trennung der gasförmigen Reaktionsprodukte mit großer Reinheit der abgetrennten Komponenten erreichen. Die Extraktionsmittel der jeweiligen Trennstufe können dabei in Bezug auf Selektivität (Maß für die Reinheit, mit dem das Extrakt aus dem Stoffgemisch abgetrennt wird) und Kapazität (Maß für die Aufnahmefähigkeit des Extraktionsmittels) optimal an die jeweilige herauszutrennende Komponente der gasförmigen Reaktionsprodukte der Dampf-Dealkylierung angepaßt werden.

Vorzugsweise werden die gasförmigen Reaktionsprodukte vor der Flüssig-Gas Extraktion einem Trocknungsverfahren zur Verringerung des Wassergehaltes unterzogen, wobei insbesondere Trocknungsverfahren unter Ausfrieren beziehungsweise Auskondensieren von Wasser, Trocknungsverfahren unter Verwendung eines Kondensatabscheiders, Molekularsiebes und/oder eines Wechselbettadsorbers mit Aluminiumgel angewendet werden. Nach der Dampf-Dealkylierung enthalten die gasförmigen Reaktionsprodukte der Dampf-Dealkylierung neben den hauptsächlichen Bestandteilen Wasserstoff, Kohlenmonoxid, Kohlendioxid und Methan noch einen Anteil gasförmigen Wassers. Durch die Trocknung der gasförmigen Reaktionsprodukte und somit der Entfernung des gasförmigen Wassers aus den gasförmigen Reaktionsprodukten wird die Schärfe der anschließenden Trennungen erhöht.

Vorteilhafterweise wird Kohlendioxid von den gasförmigen Reaktionsprodukten der Dampf-Dealkylierung mittel Flüssig-Gas Extraktion abgetrennt.

In einer bevorzugten Ausgestaltung der Erfindung erfolgt die Trennung von Kohlendioxid von den restlichen gasförmigen Reaktionsprodukten vor einem mehrstufigen Verdichtungsverfahren oder aus einer beliebigen Verdichterstufe heraus. Durch die Abtrennung von Kohlendioxid aus den gasförmigen Reaktionsprodukten vor dem mehrstufigen Verdichtungsverfahren oder aus einer beliebigen Verdichterstufe heraus wird das Volumen des zu verdichtenden Gasstroms erheblich reduziert, wodurch eine Energieeinsparung für die Verdichtung von bis zu 55% erreicht werden kann.

In einer anderen Ausgestaltung der Erfindung erfolgt die Trennung von Kohlendioxid von den restlichen gasförmigen Reaktionsprodukten nach einem mehrstufigen Verdichtungsverfahren. Nach dem mehrstufigen Verdichtungsverfahrens ist der Partialdruck des Kohlendioxids deutlich höher, wodurch die Trennung mit einer deutlich kleineren Menge an ionischer Flüssigkeit durchgeführt werden kann. In Anlagen mit einem kleinen Kohlendioxidanteil in den gasförmigen Reaktionsprodukten überwiegt bei dieser Ausgestaltung der Erfindung die Kosteneinsparung durch die Einsparung an ionischer Flüssigkeit gegenüber der aufgrund des kleinen Volumenanteils geringen Energieminimierung bei der Verdichtung.

Vorteilhafterweise werden die kohlendioxidfreien gasförmigen Reaktionsprodukte in ein Verfahren zur Wassergas-Shift-Reaktion geführt. In einem Verfahren zur Wassergas-Shift-Reaktion wird das in den gasförmigen Reaktionsprodukten vorhandene Kohlenmonoxid mittels Wasser in Kohlendioxid und Wasserstoff umgewandelt. Durch die vorherige Abtrennung von Kohlendioxid wird das Reaktionsgleichgewicht zugunsten der Reaktionsprodukte verschoben und eine erhöhte Menge des Wertproduktes Wasserstoff gebildet.

Zweckmäßigerweise wird in den gasförmigen Reaktionsprodukten noch vorhandenes Kohlenmonoxid von den gasförmigen Reaktionsprodukten getrennt. Das von den gasförmigen Reaktionsprodukten abgetrennte Kohlenmonoxid wird als Einsatzstoff in ein Verfahren zu einer Shift-Reaktion unter Gewinnung von Wasserstoff und/oder als Einsatzstoff in den Eingangsbereich der Vorrichtung zur Dampf-Dealkylierung geführt. Bei einer Verwendung des abgetrennten Kohlenmonoxids als Einsatzstoff in einem Verfahren zur Shift-Reaktion kann ebenfalls die Menge des gebildeten Wertproduktes Wasserstoff erhöht werden. Wird Kohlenmonoxid in den Eingangsbereich der Vorrichtung zur Dampf-Dealkylierung geführt, kann es sich positiv auf unerwünschte Nebenreaktionen der Dampf-Dealkylierung, wie zum Beispiel die vollständige Vergasung der Aromaten, auswirken.

Als ebenso zweckmäßig erweist sich die Abtrennung von Methan und anderer leichter Alkane aus den gasförmigen Reaktionsprodukten. Das von den gasförmigen Reaktionsprodukten abgetrennte Methan und die anderen leichten Alkane können vorteilhafterweise als Brennstoff in der Anlage, bevorzugt als Brennstoff zur Erzeugung der zur Dampf-Dealkylierung benötigten Reaktionswärme, verwendet werden.

Bevorzugt wird von den verdichteten gasförmigen Reaktionsprodukten Wasserstoff mittels eines Druckwechselabsorptionsverfahrens abgetrennt. Wasserstoff wird als weiteres Wertprodukt des Verfahrens gewonnen und kann vorteilhaft als Einsatzstoff in den Eingangsbereich der Vorrichtung zur Dampf-Dealkylierung, als Einsatzstoff in ein Wasserstoff verbrauchendes Verfahren der Anlage und/oder als Wertprodukt aus der Anlage geführt werden.

Mit der vorliegenden Erfindung gelingt es insbesondere, die gasförmigen Reaktionsprodukte der Dampf-Dealkylierung auf einfache Weise mit hoher Reinheit zu trennen.

Im Folgenden soll die Erfindung anhand der grafischen Darstellung eines Ausführungsbeispiels der Erfindung näher erläutert werden.

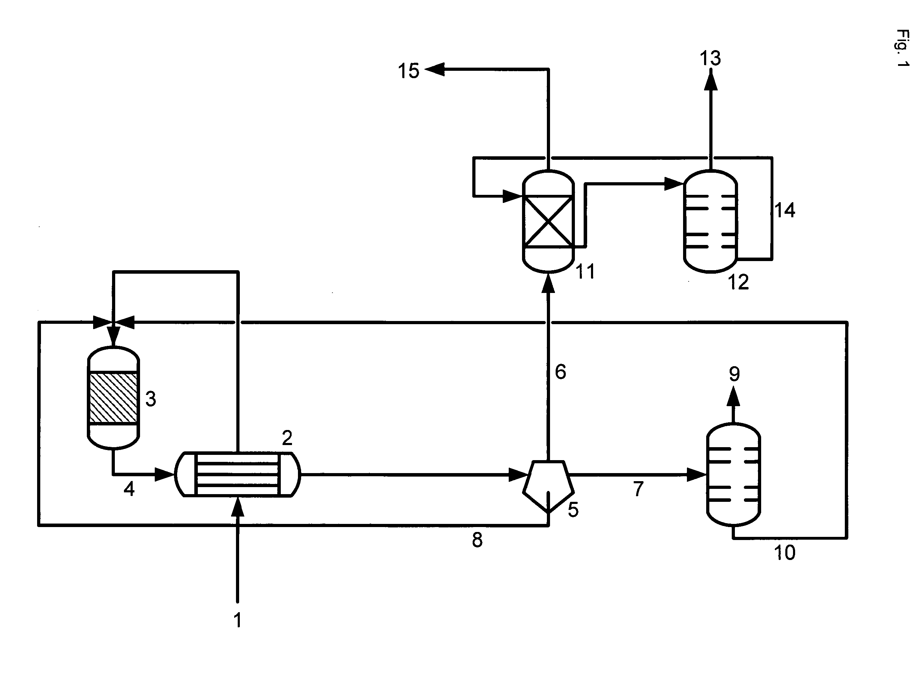

Figur 1 zeigt das Verfahrensschema einer Ausgestaltung der Erfindung. Die jeweilige Kohlenwasserstofffraktion (C₆₊-Fraktion oder C₇₊-Fraktion) (1) wird über einen Wärmetauscher (2) als Einsatzstoff in ein Verfahren zur Dampf-Dealkylierung (3) geführt. Die Reaktionsprodukte der Dampf-Dealkylierung (4) werden über den Wärmetauscher (2), der gleichzeitig die Kohlenwasserstofffraktion (1) vorwärmt, und einen weiteren Kühler (nicht dargestellt) abgekühlt und zu einem 3-Phasenseparator (5) geführt. Dort werden die gasförmigen Reaktionsprodukte (6) von den Kohlenwasserstoffen (7) und Wasser (8) getrennt. Aus den Kohlenwasserstoffen (7) wird das gewünschte Wertprodukt Benzol (9) abgetrennt, während die restlichen Kohlenwasserstoffe (10) ebenso wie das Wasser (8) erneut in den Einsatz zur Dampf-Dealkylierung gefahren werden. Die gasförmigen Reaktionsprodukte werden in ein Verfahren zur Abtrennung von Kohlendioxid mittels Flüssig-Gas-Extraktion mit einer ionischen Flüssigkeit als Extraktionsmittel geführt. Die ionische Flüssigkeit wird mit Kohlendioxid beladen (11) und anschließend zur Regeneration (12) geführt. Dort wird die ionische Flüssigkeit erwärmt, so dass Kohlendioxid aus dem Extraktionsmittel entweicht und als Produkt aus der Anlage geführt wird (13). Die regenerierte Flüssigkeit (14) wird zurück zur Beladung geführt. Die nunmehr kohlendioxidfreien, überwiegend Kohlenmonoxid, Wasserstoff und Methan enthaltenden gasförmigen Reaktionsprodukte (15) werden zu einem Verfahren zur Abtrennung von Wasserstoff geführt. Als Wertprodukte werden somit aus den gasförmigen Reaktionsprodukten Kohlendioxid und Wasserstoff gewonnen.

## Patentansprüche

1. Verfahren zur Trennung der gasförmigen Reaktionsprodukte der Dampf-Dealkylierung einer Kohlenwasserstofffraktion mit mindestens sechs Kohlenstoffatomen (C₆₊-Fraktion) und/oder einer Kohlenwasserstofffraktion mit mindestens sieben Kohlenstoffatomen (C₇₊-Fraktion) in einer Anlage zur Erzeugung von Kohlenwasserstoffen aus kohlenwasserstoffhaltigem Einsatz, wobei als Reaktionsprodukte der Dampf-Dealkylierung aromatische Kohlenwasserstoffe und entsprechende Alkane neben gasförmigen Reaktionsprodukten vorwiegend bestehend aus Wasserstoff, Kohlenmonoxid, Kohlendioxid und Methan entstehen, **dadurch gekennzeichnet, dass** die Trennung der gasförmigen Reaktionsprodukte mittels Flüssig-Gas Extraktion erfolgt, wobei als Extraktionsmittel eine Flüssigkeit eingesetzt wird, die zumindest eine ionische Flüssigkeit enthält oder aus einer oder mehreren ionischen Flüssigkeiten besteht und das Extraktionsmittel zuerst mit der herauszutrennenden Komponente beladen und anschließend unter Freisetzung der herauszutrennenden Komponente regeneriert wird.

2. Verfahren nach Anspruch 1, **dadurch gekennzeichnet, dass** jede ionische Flüssigkeit, die Bestandteil des Extraktionsmittels ist, aus der Menge der folgenden ionischen Flüssigkeiten gewählt wird, für welche gilt: lonische Flüssigkeiten sind Substanzen der allgemeinen Formel aA<m+> bB<n->, wobei a und b ganze Zahlen sind und m=1 oder m=2 und n=1 oder n=2 und a*m=b*n ist und das verwendete Kation A ausgewählt ist aus:
a) quarternären Ammonium-Kationen der allgemeinen Formel [NRR¹R²R³]⁺,
b) quarternäre Phosphonium-Kationen der allgemeinen Formel [PRR1 R2R3]⁺,
c) Imidazolium-Kationen 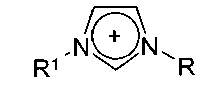 wobei der Imidazol-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
d) Pyridinium-Kationen 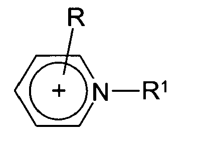 wobei der Pyridin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
e) Pyrrolidinium-Kation 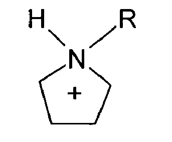 wobei der Pyrrolidin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
f) Pyrazolium-Kation 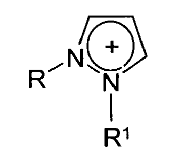 wobei der Pyrazol-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
g) Triazolium-Kation 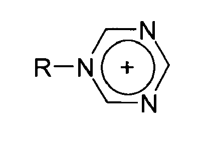 wobei der Triazol-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitrit, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
h) Pyrimidinium-Kation 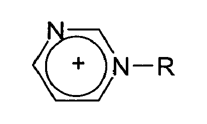 wobei der Pyrimidin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
i) Chinolin-Kation 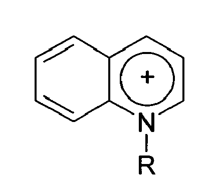 wobei der Chinolin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel-(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
j) Isochinolin-Kation 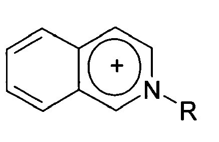 wobei der Isochinolin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
k) Morpholinium-Kation 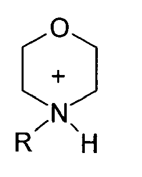 wobei der Morpholin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
l) Oxazolium-Kation 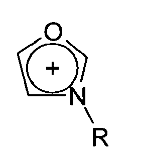 wobei der Oxazol-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
m) Isoxazolium-Kation 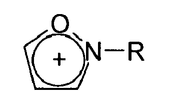 wobei der Isoxazol-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
n) Thiazoliumkation 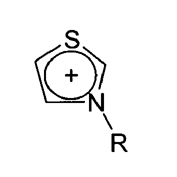 wobei der Thiazol-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
o) Guanidiniumkation 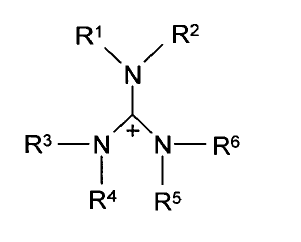 wobei der Guanidin-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
p) Isouroniumkation 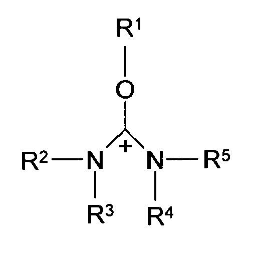 wobei der lsouronium-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
q) Isothiouroniumkation 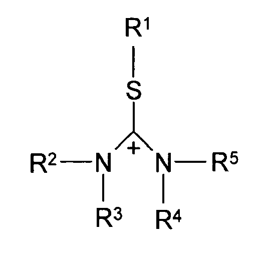
wobei der Isothiouronium-Kern substituiert sein kann durch wenigstens eine Gruppe, die ausgewählt ist aus Halogeniden, Hydroxyl, linearen oder verzweigten substituierten Alkylresten bis 20 Kohlenstoffatomen, die mit einer oder mehreren Gruppen ausgewählt aus den Halogeniden, Hydroxyl, Nitril, Amin, Thiol substituiert sein können, C₅-C₁₂-Aryl- oder C₅-C₁₂-Aryl-C₁-C₆-Alkylgruppen, Resten der allgemeinen Formel -(R-X)n-R' mit n=1-10, wobei R eine lineare oder verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist;
und in den allgemeinen Formeln die Reste R, R¹, R², R³ unabhängig voneinander ausgewählt sind aus Wasserstoff, Halogeniden, Hydroxyl, linearen oder verzweigten substituierten oder unsubstituierten Alkylresten bis 20 Kohlenstoffatomen, die mit einer oder mehreren Gruppen ausgewählt aus den Halogeniden, Hydroxyl, Nitril, Amin, Thiol substituiert sein können;
Heteroaryl-, Heteroaryl-C₁-C₆-Alkylgruppen mit 3 bis 8 Kohlenstoffatomen im Heteroarylrest und wenigstens einem Heteroatom ausgewählt aus N, O und S, der mit wenigstens einer Gruppe ausgewählt aus C₁-C₆-Alkylgruppen und/oder Halogenatomen substituiert sein können;
Aryl-, Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenatom substituiert sein können;
Resten der allgemeinen Formel -(R-X)n-R' mit n=1-10, wobei R eine lineare oer verzweigte Alkylgruppe mit 1 bis 20 Kohlenstoffatomen ist, R' Wasserstoff oder eine lineare oder verzweigte Alkylgruppe ist und X eine Ethergruppe, Thioethergruppe, Estergruppe, Siloxangruppe oder Amidgruppe ist.
Als Anionen sind alle Anionen anwendbar, wobei das verwendete Anion B vorzugsweise ausgewählt ist aus:
[PF₆]⁻, [BF₄]⁻, [CF₃CO₂]⁻, [CF₃SO₃]⁻, [(CF₃SO₂)₂N]⁻, [(CF₃SO₂)(CF₃COO)N]⁻, [R⁴-SO₃]⁻, [R⁴-O-SO₃]⁻, [R⁴-COO]⁻, Cl⁻, Br⁻, I⁻, [NO₃]⁻, [N(CN)₂]⁻, [HSO₄]⁻, [R⁴R⁵PO₄]⁻, [CN]⁻, [SCN]⁻, [ClO₃]⁻, [ClO₄]⁻
und die Reste R⁴ und R⁵ unabhängig voneinander ausgewählt sind aus der Gruppe bestehend aus Wasserstoff, linearen oder verzweigten, gesättigten oder ungesättigten, aliphatischen oder alicyclischen Alkylgruppen mit 1 bis 20 Kohlenstoffatomen; Heteroaryl-, Heteroaryl-Cl-C6-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C1-C6-Alkylgruppen und/oder Halogenatomen substituiert sein können; Aryl-, Aryl-C1-C6-Alkylgruppen mit 5 bis 12 Kohlenstoffatomen im Arylrest, die mit wenigstens einer C1-C6-Alkylgruppe und/oder einem Halogenatom substituiert sein können.

3. Verfahren nach Anspruch 1 oder 2, **dadurch gekennzeichnet, dass** die Trennung der gasförmigen Reaktionsprodukte im Gleichstrom oder im Gegenstrom mit dem Extraktionsmittel oder durch Durchperlen des Extraktionsmittels erfolgt.

4. Verfahren nach einem der Ansprüche 1 bis 3, **dadurch gekennzeichnet, dass** die Regeneration des Extraktionsmittels mittels Druckreduktion, Anlegung eines Unterdrucks oder Vakuums und/oder mittels Temperaturerhöhung des Extraktionsmittels erfolgt.

5. Verfahren nach einem der Ansprüche 1 bis 4, **dadurch gekennzeichnet, dass** mindestens zwei Vorrichtungen zur Beladung und Regeneration verwendet werden, wobei immer in mindestens einer Vorrichtung die Beladung und in mindestens einer Vorrichtung die Regeneration des Extraktionsmittels erfolgt.

6. Verfahren nach einem der Ansprüche 1 bis 5, **dadurch gekennzeichnet, dass** mindestens zwei Verfahren zur Flüssig-Gas Extraktion nacheinander unter Verwendung unterschiedlich zusammengesetzter Extraktionsmittel durchgeführt werden.

7. Verfahren nach einem der Ansprüche 1 bis 6, **dadurch gekennzeichnet, dass** die gasförmigen Reaktionsprodukte vor der Flüssig-Gas Extraktion einem Trocknungsverfahren zur Verringerung des Wassergehaltes unterzogen werden, wobei insbesondere Trocknungsverfahren unter Ausfrieren beziehungsweise Auskondensieren von Wasser, Trocknungsverfahren unter Verwendung eines Kondensatabscheiders, Molekularsiebes und/oder eines Wechselbettadsorbers mit Aluminiumgel angewendet werden.

8. Verfahren nach einem der Ansprüche 1 bis 7, **dadurch gekennzeichnet, dass** Kohlendioxid von den gasförmigen Reaktionsprodukten der Dampf-Dealkylierung mittels Flüssig-Gas Extraktion abgetrennt wird.

9. Verfahren nach Anspruch 8, **dadurch gekennzeichnet, dass** die Trennung von Kohlendioxid von den restlichen gasförmigen Reaktionsprodukten vor einem mehrstufigen Verdichtungsverfahren oder aus einer beliebigen Verdichterstufe heraus erfolgt.

10. Verfahren nach Anspruch 8, **dadurch gekennzeichnet, dass** die Trennung von Kohlendioxid von den restlichen gasförmigen Reaktionsprodukten nach einem mehrstufigen Verdichtungsverfahren erfolgt.

11. Verfahren nach Anspruch 9 oder 10, **dadurch gekennzeichnet, dass** die kohlendioxidfreien gasförmigen Reaktionsprodukte in Verfahren zur Wassergas-Shift-Reaktion geführt werden.

12. Verfahren nach einem der Ansprüche 1 bis 11, **dadurch gekennzeichnet, dass** Kohlenmonoxid von den gasförmigen Reaktionsprodukten getrennt wird.

13. Verfahren nach Anspruch 12, **dadurch gekennzeichnet, dass** von den gasförmigen Reaktionsprodukten getrenntes Kohlenmonoxid als Einsatzstoff in ein Verfahren zu einer Shift-Reaktion unter Gewinnung von Wasserstoff und/oder als Einsatzstoff in den Eingangsbereich der Vorrichtung zur Dampf-Dealkylierung geführt wird.

14. Verfahren nach einem der Ansprüche 1 bis 3, **dadurch gekennzeichnet, dass** Methan und anderer leichter Alkane von den gasförmigen Reaktionsprodukten getrennt wird.

15. Verfahren nach Anspruch 14, **dadurch gekennzeichnet, dass** von den verdichteten gasförmigen Reaktionsprodukten Wasserstoff mittels eines Druckwechselabsorptionsverfahrens abgetrennt wird.
